# EUROPÄISCHE PATENTANMELDUNG

(11) **EP 4 781 951 A1**
(43) Veröffentlichungstag der Anmeldung: **29.07.2026**
(21) Anmeldenummer: 25154163.7
(22) Anmeldetag: 27.01.2025
(51) Int. Cl.: A61F 2/50, A61F 2/76, A61F 2/78

(54) **VERFAHREN ZUM HERSTELLEN EINES MEHRSCHICHTIGEN HTV SILIKONKÖRPERS, VERFAHREN ZUM HERSTELLEN EINES MEHRSCHICHTIGEN SILIKONLINERS, MEHRSCHICHTIGER HTV SILIKONKÖRPER, MEHRSCHICHTIGER SILIKONLINER UND SYSTEM ZUR SENSORISCHEN RÜCKMELDUNG**

(71) Anmelder: SiliCore GmbH, 8306 Brüttisellen (CH)
(72) Erfinder: Markwalder, Martin, 8306 Brüttisellen (CH); von Essen, Stefan, 14943 Luckenwalde (DE); vo Essen, Tobias, 15806 Glienick/Zossen (DE)
(74) Vertreter: Gulde & Partner

(57) **Zusammenfassung**

Die Erfindung betrifft ein Verfahren zum Herstellen eines mehrschichtigen HTV Silikonkörpers (100) und einen daraus herstellbaren mehrschichtigen HTV Silikonkörper (100) sowie ein Verfahren zum Herstellen eines mehrschichtigen Silikonliners (200) und einen daraus herstellbaren mehrschichtigen Silikonliner (200). Ferner betrifft die Erfindung ein System (10) zur sensorischen Rückmeldung.

Es ist vorgesehen, dass ein mehrschichtiger HTV Silikonkörper (100) für eine Prothese oder einen Überzug der Prothese eine zwischen zwei HTV-Silikonschichten eingebettete Sensoreinheit (102, 104) zum Erfassen einer Temperatur und/oder eines Drucks umfasst. Derart erfasste Temperatur- und/oder Druckwerte werden an eine in einem mehrschichtigen Silikonliner (200) eingebettete Aktoreinheit (202, 204) zum Erzeugen einer Temperatur und/oder eines entsprechenden Drucks übermittelt. Die Aktoreinheit (202, 204) ermöglicht dem Träger der Prothese oder des Überzugs der Prothese durch den an seinem Stumpf getragenen mehrschichtigen Silikonliner (200), auf die Prothese einwirkende Temperaturen und/oder Drücke an den Stumpf im Sinne einer sensorischen Rückmeldung weiterzuleiten.

## Beschreibung

Die Erfindung betrifft ein Verfahren zum Herstellen eines mehrschichtigen HTV Silikonkörpers und einen daraus herstellbaren mehrschichtigen HTV Silikonkörper sowie ein Verfahren zum Herstellen eines mehrschichtigen Silikonliners und einen daraus herstellbaren mehrschichtigen Silikonliner. Ferner betrifft die Erfindung ein System zur sensorischen Rückmeldung.

Wenn ein Mensch mit einer Amputation versorgt wird, hängt diese von den entsprechenden Umständen und Bedürfnissen der Person ab. Um fehlende oder eingeschränkte Körperteile zu ersetzen oder zu vervollständigen werden bekanntermaßen Prothesen eingesetzt. Zur Herstellung einer Prothese wird beispielsweise zunächst ein grober Prothesenrohling erstellt, der durch einen Orthopädietechniker an die Form und Gestalt des betroffenen Körperteils sukzessive und individuell angepasst wird. Der Patient begibt sich häufig mehrmals zum Orthopädietechniker, um die individuell angefertigte Prothese abzustimmen.

Wenn eine lebensechte, individuelle Kosmetik gewünscht ist, egal ob als kosmetischer Überzug für eine Prothese, beispielsweise für eine Hand- oder Armprothese, oder als Habitusprothese, ist es wünschenswert hoch-temperatur vernetzende (High Temperature Vulcanizing - HTV) Silikone zu verwenden. HTV Silikone sind bevorzugt Gummi mit hoher Konsistenz (High Consistency Rubber - HCR), wärmegehärtetes Gummi (Heat Cured Rubber) oder HTV Silikonkautschuk. Anders ausgedrückt ist ein HTV Silikon (vor dem Vulkanisierungsprozess) ein bevorzugt knetbares Silikon. Bei HTV Silikonen handelt es sich um eine bestimmte Art von Silikon, die sich durch exzellente mechanische Eigenschaften, wie hohe Bruchdehnung und Weiterreißfestigkeit auszeichnet. Etwas, das bei einer täglich getragenen Prothese, die hohen Belastungen ausgesetzt ist, unbedingt notwendig ist, um eine adäquate Lebensdauer zu gewährleisten. Es wird vermutet, dass das knetbare (pastöse) HTV Silikon deshalb so gute physikalische Eigenschaften hat, weil hauptsächlich Kieselsäure als Füllstoff enthalten ist. Die feinen Partikel der Kieselsäure sorgen für eine mechanische Verstärkung des Silikons, indem sie die Polymerketten stützen. Dies führt dazu, dass der Kautschuk eine feste, aber formbare (knetbare) Konsistenz aufweist.

Der große Nachteil von HTV Silikonen liegt darin, dass diese, entgegen anderer Silikone, wie kaltvernetzende (Raumtemperatur vernetzend - RTV) Silikone oder Flüssig-Silikone (*Liquid Silicone Rubber* - LSR), als Feststoff verarbeitet werden müssen. Üblicherweise werden beide zur Herstellung eines HTV-Silikons erforderlichen Komponenten (zusammen mit bestimmten Farbpigmenten) in einer Walze gemischt, ausgewalzt und dann auf einen Gipskörper modelliert. Dementsprechend ist ein Unterscheidungsmerkmal zwischen LSR Silikonen und HCR ist die fließfähige oder flüssige Beschaffenheit von LSR-Materialien.

Dieser Feststoff-Arbeitsprozess stellt enorme Limitierungen dar: Eine dünne Wandstärke lässt sich nur bis zu einem gewissen Grad ermöglichen, die farbliche Gestaltung erfordert das "Einkneten" unterschiedlich eingefärbter Silikonteile, die künstlerisch-handwerkliche Fähigkeit des Technikers ist maßgeblich entscheidend über den lebensechten Charakter der Prothese oder des Prothesenüberzugs, und es wird durch diesen Arbeitsprozess immer nur ein Einzelstück gefertigt.

Jedoch kann eine noch so lebensecht aussehende Prothese die sensorischen Fähigkeiten des fehlenden oder eingeschränkten Körperteils nicht ersetzen. Aufgrund der hohen Mindestwandstärke der ausgewalzten HTV-Silikone gestaltet es sich schwierig, Messsignale von an der Außenseite der Prothese wirkenden physikalischen Größen, wie Kraft, Druck, Temperatur oder dergleichen, durch das ausgewalzte HTV Silikon hindurch an der Innenseite der Prothese durch entsprechende Sensoren zu erfassen. Auf der Außenseite angeordnete Sensoren würden das lebensechte Aussehen der Prothese erheblich beeinträchtigen. Insofern wird einem Träger einer Prothese stets bewusst sein, dass es sich bei der Prothese um einen Fremdkörper handelt, wodurch die Akzeptanz der Prothese für ihren Träger geschmälert wird. Eine gesteigerte Akzeptanz der Prothese für ihren Träger ist nicht nur wünschenswert, um das allgemeine Wohlbefinden des Trägers der Prothese zu verbessern, sondern auch um das mit dem Verlust oder der Einschränkung des Körperteils verbundene Trauma besser bewältigen zu können.

Ferner beklagen Träger von Prothesen vermehrt das Auftreten von Phantomschmerzen. Es wird vermutet, dass sich Phantomschmerzen durch komplexe Prozesse im Gehirn des Trägers ergeben, die durch die fehlende Stimulierung betreffender Gehirnareale aufgrund der ausbleibenden Reizsignale des fehlenden oder eingeschränkten Körperteils hervorgerufen werden.

Daher wäre es wünschenswert, (lebensechte) Prothesen aus Silikon herzustellen, die die sensorischen Fähigkeiten des fehlenden oder eingeschränkten Körperteils zumindest teilweise ersetzen können, insbesondere auch um das Auftreten von Phantomschmerzen zu reduzieren.

Der Erfindung liegt daher nun die Aufgabe zugrunde, eine Prothese für ein fehlendes oder eingeschränktes Körperteil mit verbesserter Funktionalität zu schaffen, welches die Akzeptanz und das Wohlbefinden ihres Trägers steigert.

Die erfindungsgemäße Aufgabe wird gelöst durch ein Verfahren zum Herstellen eines mehrschichtigen HTV Silikonkörpers für eine Prothese oder einen Überzug der Prothese und einen daraus herstellbaren mehrschichtigen HTV Silikonkörper, ein Verfahren zum Herstellen eines mehrschichtigen Silikonliners und einen daraus herstellbaren mehrschichtigen Silikonliner und System zur sensorischen Rückmeldung umfassend den mehrschichtigen HTV Silikonkörper und den mehrschichtigen Silikonliner gemäß den unabhängigen Patentansprüchen. Bevorzugte Weiterbildungen sind Gegenstand der jeweils rückbezogenen Unteransprüche.

Ein erster Aspekt betrifft ein Verfahren zum Herstellen eines mehrschichtigen HTV Silikonkörpers für eine Prothese oder einen Überzug der Prothese. Gemäß der vorliegenden Offenbarung umfasst der Begriff Prothese bevorzugt die vollständige Prothese oder nur eines Teiles davon. Der Begriff Überzug umfasst bevorzugt den vollständigen Überzug oder nur einen Teil davon. Mit anderen Worten ist das Verfahren ein Verfahren zur Herstellung eines mehrschichtigen HTV Silikonkörpers für eine Prothese oder eines Teiles davon bzw. für einen Überzug für eine Prothese oder einen Teil davon. Die hergestellte Prothese oder der Überzug dafür dienen insbesondere zum Ersatz eines fehlenden oder eingeschränkten Körperteils, beispielsweise eines Fingers, einer Hand, eines Arms, eines Beins, eines Fußes, eines Ohres und dergleichen oder auch nur eines Teiles davon, beispielsweise eine Teilhand oder ein Teilfuß.

Gemäß dem Verfahren wird eine erste HTV-Silikonschicht (des mehrschichtigen HTV Silikonkörpers) basierend auf einer HTV Silikon-haltigen Lösung und einer Form der Prothese oder des Überzugs hergestellt. Das HTV Silikon der HTV Silikon-haltigen Lösung ist hierbei in einem Lösungsmittel gelöst. Mit anderen Worten wird ein verdünntes, also in einen flüssigem bzw. fließfähigem Zustand überführtes, HTV-Silikon, also ein Festsilikonkautschuk, welches normalerweise als Festkörper verarbeitet wird, verwendet und als Fluid oder Lösung in flüssigem bzw. fließfähigem Zustand verarbeitet. Es wurde festgestellt, dass die Verarbeitung von HTV Silikon als HTV Silikon-haltige Lösung eine vereinfachte Verarbeitung des HTV Silikons ermöglicht, ohne die (gewünschten) mechanischen Eigenschaften des HTV Silikons im vulkanisierten Endprodukt zu verändern bzw. zu verlieren. Insofern wurde erkannt, dass vor allem das Verflüssigen von HTV Silikonen einen Einsatz von HTV Silikonen in Bereichen ermöglicht, die zuvor nicht mit HTV-Silikonen aufgrund ihrer Feststoff-Eigenschaft abgedeckt werden konnten, und insofern bislang den im flüssigen Zustand verarbeiteten LSR-Silikone vorbehalten war. Auch andere potentielle Anwendungslösungen sind damit denkbar. Der Begriff HTV Silikon umfasst bevorzugt keine LSR Silikone. Mit anderen Worten fallen LSR Silikone bevorzugt nicht unter den Begriff HTV Silikon und sind somit explizit ausgeschlossen. Pastöse HTV Silikone sind im Vergleich zu LSR Silikonen teurer, sodass bei Anwendungen mit "Flüssigsilikonen" in der Fachwelt automatisch zu einem LSR Silikon gegriffen wird. Darüber hinaus steigt das Volumen der HTV Silikon-haltigen Lösung beim Lösen des HTV Silikons um bis zu einem 3fachen an. In der Fachwelt wurde bislang davon abgesehen, pastöse HTV Silikone mit einem Lösungsmittel zu lösen, da davon ausgegangen wurde, dass das HTV Silikon durch den hohen Anteil des Lösungsmittels Schaden nimmt, sodass es im anschließenden Vulkanisierungsprozess zu einer Blasenbildung kommt. Durch die Verwendung der HTV Silikon-haltigen Lösung können HTV Silikonschichten mit Schichtdicken (Wandstärken) hergestellt werden, die erheblich geringer sind als die Wandstärken, die mit den konventionellen Festkörper-Verarbeitungsschritten von HTV Silikonen erreicht werden können.

Ferner wird mindestens eine zum Erfassen einer Temperatur und/oder eines Drucks eingerichtete Sensoreinheit auf der ersten HTV-Silikonschicht (des mehrschichtigen HTV Silikonkörpers) angeordnet. Beispiele für die Sensoreinheit sind elektronische Bauteile, wie temperatur-sensitive, elektrische/elektronische/thermoelektrische Bauteile, beispielsweise temperaturabhängige elektrische Widerstände, auf dem Seebeck-Effekt-basierte Bauteile, oder Platin- oder NTC-(Negative Temperature Coefficient Thermistor)Halbleiter-Temperatursensoren, die über einen Messstrom angeregt werden und deren elektrischer Widerstand auf vorhersagbare Weise von der anliegenden Temperatur abhängt, und druck-sensitive, elektrische/elektronische Bauteile, beispielsweise druckabhängige elektrische Widerstände, auf dem Piezoeffekt-basierte Bauteile oder Silikon-basierte, elastisch verformbare Widerstände zwischen zwei elektrischen Kontakten auf Flex-Leiterbahnen, die durch Druckeinwirkung ihren elektrischen Widerstand auf vorhersehbare Weise verändern. Eine Messfläche der Sensoreinheit umfasst bevorzugt eine Fläche von 1 cm² bis 10 cm², vorzugsweise 2 cm² bis 8 cm² , besonders bevorzugt 4 cm² bis 6 cm², beispielsweise 5 cm². Beispielhafte Größenordnungen der erfassbaren Drücke liegen bevorzugt in der Größenordnung von 100 g (0,1 N) bis 5 kg (5 N) im Falle für eine Handprothese. Beispielhafte Größenordnungen der erfassbaren Temperaturen sind im Bereich von 0 °C bis 50°C. Es ist verständlich, dass die vorliegende Offenbarung nicht auf die genannten Größenordnungen beschränkt ist und die erfassbaren Parameter in anderen Größenordnungen liegen können, insbesondere abhängig von dem von der Prothese zu ersetzenden Körperteil. Bevorzugt wird eine Vielzahl von zum Erfassen einer Temperatur und/oder eines Drucks eingerichteten Sensoreinheiten auf der ersten HTV-Silikonschicht verteilt angeordnet. Mit anderen Worten kann die Anzahl und der jeweilige Ort der Sensoreinheiten an die jeweils vorliegende Prothese individuell gestaltet werden, um die als relevanten betrachteten Bereiche der Prothese mit den Sensoreinheiten abzudecken. Die Verwendung von mehreren Sensoreinheiten ermöglicht folglich die (ortsselektive) Erfassung von Temperaturen und/oder Drücken bezogen auf unterschiedliche (lokale) Bereichen des mehrschichtigen HTV Silikonkörpers.

In einem weiteren Schritt des Verfahrens wird eine zweite HTV-Silikonschicht (des mehrschichtigen HTV Silikonkörpers) auf der Sensoreinheit und der ersten HTV-Silikonschicht basierend auf der HTV Silikon-haltigen Lösung hergestellt. Mit anderen Worten wird die Sensoreinheit zwischen die erste HTV-Silikonschicht und der zweiten HTV-Silikonschicht eingebettet. Folglich wird ein mehrschichtiger HTV Silikonkörper für die Prothese oder des Überzuges der Prothese ausgebildet. Durch den mehrschichtigen Aufbau ist es möglich, elektronische Bauteile und andere Komponenten zwischen den HTV-Silikonschichten anzuordnen und nach außen hin zu verstecken beziehungsweise abzuschirmen. Hierdurch ist die Sensoreinheit verdeckt, geschützt und in ausreichender Nähe zu der Außenseite des Silikonkörpers angeordnet, um Messsignale von auf die Außenseite der Prothese einwirkenden Temperaturen und/oder Drücken in guter Qualität bereitstellen zu können. Somit wird eine lebensechte Prothese aus Silikon hergestellt, die die sensorischen Fähigkeiten des fehlenden oder eingeschränkten Körperteils zumindest hinsichtlich der Temperatur und/oder des Druckes zumindest teilweise ersetzen kann. Ferner können mehrschichtige HTV Silikonkörper in ihren Eigenschaften variabler gestaltet werden. Beispielsweise kann eine Schichtdicke und/oder ein Schichtmaterial der einzelnen Schichten unterschiedlich ausgebildet werden und/oder zwischen den Schichten verschiedene Materialen - nebst der Sensoreinheit - eingebracht bzw. eingebettet werden.

Bevorzugt werden unterschiedliche HTV Silikon-haltige Lösungen verwendet, insbesondere zur Ausbildung der verschiedenen HTV-Silikonschichten des mehrschichtigen HTV Silikonkörpers. Bevorzugt werden HTV Silikon-haltige Lösungen mit HTV Silikonen unterschiedlicher Shore-Härte eingesetzt und/oder HTV Silikon-haltige Lösungen versetzt mit unterschiedlichen Farbpigmenten verwendet. Bevorzugt wird ein Auftragen der ersten HTV-Silikonschicht und der letzten HTV-Silikonschicht des mehrschichtigen HTV Silikonkörpers derart gewählt, dass die mechanischen Eigenschaften dieser HTV-Silikonschichten, also der äußeren Schichten des mehrschichtigen HTV Silikonkörpers, von den mechanischen Eigenschaften der oder den übrigen Schichten des mehrschichtigen HTV Silikonkörpers, also den inneren bzw. innenliegenden Schichten des mehrschichtigen HTV Silikonkörpers, unterscheiden. Bevorzugt wird eine oder beide der jeweils außenliegenden Schichten des mehrschichtigen HTV Silikonkörpers aus einem HTV Silikon mit einer größeren Shore Härte und/oder mit einer größeren Schichtdicke ausgebildet. Somit kann die Widerstandskraft und somit die Lebensdauer des mehrschichtigen HTV Silikonkörpers erhöht werden und gleichzeitig eine ausreichende Flexibilität durch die dünneren bzw. weicheren innenliegenden Schichten des mehrschichtigen HTV Silikonkörpers erreicht werden.

Bevorzugt wird die Form der Prothese oder des Überzugs mittels eines 3D-Druckverfahrens oder mittels eines Abdrucks hergestellt. Gemäß der ersten Alternative wird bevorzugt von dem Patienten ein digitales 3D-Abbild des gesunden Körperteils, insbesondere als High-Density Mesh, erstellt. Dies erfolgt bevorzugt durch (direktes) Abscannen von der Haut beziehungsweise des entsprechenden Körperteils mittels eines 3D-Scanners und/oder eines bildgebenden Verfahrens, wie der Computertomographie (CT), der digitalen Volumentomographie (DVT) und dergleichen. Bevorzugt wird das digitale 3D-Abbild gespiegelt, um die Chiralität von paarig auftretenden Körperteilen zum Zwecke der Gestaltung der Form auszunutzen. Hierdurch kann eine aufwändige Gestaltung der Form vermieden werden. Außerdem wird eine individuelle Prothese erzeugt, die dem eigenen Körper des Patienten nachgeahmt ist, was eine psychologische Stütze für den Patienten darstellen kann, insbesondere bei einer erst kürzlichen erfolgten (Teil-)Amputation des Körperteils. Das digitale 3D-Abbilid wird bevorzugt unter Verwendung eines 3D-Druckers in die gegenständliche Form überführt. Bevorzugt wird das digitale 3D-Abbild zuvor noch individuell an die Wünsche des Patienten angepasst. Gemäß der zweiten Alternative erfolgt die Herstellung der Form bevorzugt über einen Silikon- oder Alginatabdruck und einen daraus erstellten Vollkörper. Auch andere Methoden, wie freies Modellieren in z.B. Ton oder Modelliermasse, sind bevorzugt vorgesehen.

Bevorzugt beträgt ein Mischungsverhältnis der HTV Silikon-haltigen Lösung 1 Teil HTV-Silikon zu 1 Teil Lösungsmittel, vorzugsweise mindestens 1 Teil HTV Silikon zu 2,5 Teilen Lösungsmittel, besonders bevorzugt bis zu 1 Teil HTV Silikon zu 4,5 Teilen Lösungsmittel. Ein bevorzugtes Lösungsmittel ist Hexamethyldisiloxan. Es wurde festgestellt, dass das HTV Silikon bei einem Mischungsverhältnis von 1 Teil HTV Silikon zu mindestens einem Teil Hexamethyldisiloxan keine der erwarteten Schäden nimmt. Jedoch kann es zu einer Blasenbildung in der HTV Silikon-haltigen Lösung im Verdungstungsprozess kommen, wenn das Mischungsverhältnis 1 Teil HTV Silikon zu weniger als 2,5 Teilen Hexamethyldisiloxan beträgt. Ab einem Mischungsverhältnis von 1 Teil HTV Silikon zu 2,5 Teilen oder mehr tritt keine Blasenbildung mehr auf. Bevorzugt beträgt das Mischungsverhältnis der HTV Silikon-haltigen Lösung von 1 Teil HTV Silikon zu 2,5 Teilen oder mehr. In diesem Mischungsverhältnis weist die HTV Silikon-haltige Lösung eine Viskosität von etwa 20 000 mPas auf. Mit anderen Worten ist die Viskosität der HTV Silikon-haltigen Lösung bevorzugt höchstens 25000 mPas, bevorzugt höchstens 22500 mPas. Besonders bevorzugt beträgt das Mischungsverhältnis 1 Teil Silikon zu 3,5 bis 4 Teilen Hexamethyldisiloxan. Hier liegt die Viskosität bei etwa 4000 -2500 mPaS. Mit anderen Worten ist die Viskosität der HTV Silikon-haltigen Lösung bevorzugt mindestes 500 mPas, vorzugsweise mindestens 1000 mPas und/oder höchstens 25000 mPas, bevorzugt höchstens 22500 mPas. Bevorzugt kann das Mischungsverhältnis von 1 Teil HTV Silikon zu >4 Teilen Hexamethyldisiloxan betragen. Jedoch ist zu beachten, dass dann die erhaltenen Schichtdicken der HTV Silikonschichten sehr dünn werden. Mit der Beschaffenheit der gewählten HTV Silikon-haltigen Lösung kann also die Wandstärke des hergestellten mehrschichtigen HTV Silikonkörpers variabel ausgestaltet werden. Aufgrund der geringeren Wandstärke des hergestellten mehrschichtigen HTV Silikonkörpers wird eine größere Flexibilität und ein geringeres Gewicht der späteren Prothese erreicht.

Das Lösungsmittel ist bevorzugt flüchtig. Die Flüchtigkeit (auch Verdunstungszahl) ist eine dimensionslose relative Kennzahl, die die Verdunstung eines Lösungsmittels beschreibt. Die Flüchtigkeit bezieht sich dabei bevorzugt auf die Raumtemperatur, etwa 20°C. Das Lösungsmittel ist bevorzugt leichtflüchtig (Verdunstungszahl kleiner als 10) oder mittelflüchtig (Verdunstungszahl 10 bis 35), gemessen nach DIN 53170. Ein flüchtiges Lösungsmittel ermöglicht es, dass sich das Lösungsmittel der in Form gegossenen oder geschwenkten HTV Silikon-haltige Lösung von alleine verflüchtigt, also das HTV Silikon sich von dem Lösungsmittel ohne merklichen Aufwand selbstständig zur Ausbildung einer HTV-Silikonschicht trennt.

Bevorzugt ist das Lösungsmittel ausgewählt aus Hexamethyldisiloxan, Octamethyltrisiloxan, weitere Lösungsmittel der Silangruppe, Kaltreiniger, Heptan, Kohlenwasserstoffe C6-C7, N-Alkane, Iso-Alkane, Cyclische Verbindungen und N-Hexana (z.B. Wasch-Benzin). Insbesondere Hexamethyldisiloxan, Heptan und Wasch-Benzin sind besonders gut geeignete Lösungsmittel für HTV Silikone, die eine besonders gute Löslichkeit des HTV Silikons bieten.

Bevorzugt ist die HTV Silikon-haltige Lösung mit weiteren Komponenten, wie beispielsweise Farbpigmenten, versehen. Dies ermöglicht eine einfachere Farbgestaltung des HTV Silikons durch Einrühren im Gegensatz zum Einwalzen, wie es bei HTV Silikonen üblicherweise erforderlich ist. Ferner bevorzugt wird die HTV Silikon-haltige Lösung gefiltert und/oder unter Anlegung eines Vakuums entlüftet, um die Reinheit der HTV Silikon-haltigen Lösung und des späteren HTV Silikons zu steigern.

Bevorzugt wird ein HTV Silikon einer beliebigen Shore-Härte verwendet, also beispielsweise Shore A 00-10 bis Shore A 80, gemessen nach DIN ISO 48-4. Folglich ist ein breites Anwendungsspektrum für die HTV Silikon-haltige Lösung gegeben.

In bevorzugter Ausgestaltung ist vorgesehen, dass die Form der Prothese oder des Überzugs eine Negativ- und/oder Positivform der Prothese oder des Überzugs umfasst. Bevorzugt wird in dem Herstellungsschritt der ersten HTV-Silikonschicht die HTV Silikon-haltige Lösung in der Negativform der Prothese oder in der Negativform des Überzugs ausgeschwenkt oder eine Positivform der Prothese oder eine Positivform des Überzugs mit der HTV Silikon-haltigen Lösung übergossen und/oder darin eingetaucht. Für das Ausschwenken, das Übergießen und/oder das Eintauchen wird jeweils ein Fluid verwendet, welches die oben genannte HTV Silikon-haltige Lösung enthält oder daraus besteht. Durch das Ausschwenken beziehungsweise Übergießen und/oder Eintauchen einer Form mit der bzw. in die HTV Silikon-haltige Lösung können HTV Silikonkörper mit Wandstärken hergestellt werden, die weit geringer sind als jene Wandstärken, die mit den konventionellen Festkörper-Verarbeitungsschritten von HTV Silikonen erreicht werden können. Folglich können mehrschichtige HTV Silikonkörper mit lebensechten Strukturen einfach und kostengünstig hergestellt werden. So können Prothesen und Überzüge für Prothesen hergestellt werden, die für jeden Patienten individuell, visuell den herkömmlichen Alternativen überlegen und sehr viel dünner sind. Durch die Verwendung von Negativ- bzw. Positivformen wird für die Herstellung einer weiteren Prothese (also jede weitere Versorgung des Patienten) etwa 70% der Arbeitszeit eingespart. Außerdem ist dieser Prozess zuverlässig und nicht (maßgeblich) abhängig von der Fähigkeit oder der Tagesform der die Prothese herstellenden Person. Bevorzugt ist es vorgesehen, dass zumindest ein, einige oder alle Herstellungsschritte der HTV-Silikonschichten unter Verwendung einer Negativ- und/oder Positivform der Prothese oder des Überzugs erfolgen. Beispielsweise erfolgt zum Ausbilden der zweiten Silikonschicht bevorzugt ein Ausschwenken der HTV Silikon-haltigen Lösung in der ersten HTV-Silikonschicht, die beispielsweise noch in der Negativform angeordnet ist, oder ein Übergießen der ersten HTV-Silikonschicht, die noch auf der Positivform angeordnet ist, mit der HTV Silikon-haltigen Lösung und/oder ein Eintauchen der ersten HTV-Silikonschicht, die noch auf der Positivform angeordnet ist, in die HTV Silikon-haltige Lösung. Dadurch, dass die erste HTV-Silikonschicht noch in oder auf der jeweiligen Form verbleibt, bleibt diese formstabil, wenn die zweite HTV-Silikonschicht auf diese angeordnet wird. Bevorzugt wird die Negativform vor dem Ausschwenken oder die Positivform vor dem Übergießen und/oder Eintauchen mit einem Trennmittel vorbehandelt. Dies vereinfacht das Entfernen beziehungsweise Entformen des HTV Silikonkörpers von oder aus der entsprechenden Form.

In weiterer bevorzugter Ausgestaltung ist vorgesehen, dass die erste HTV-Silikonschicht und die darauf angeordnete zweite HTV-Silikonschicht mit der dazwischen angeordneten Sensoreinheit unter Wärmezufuhr vulkanisiert wird. Mit anderen Worten werden die erste HTV-Silikonschicht und die zweite HTV-Silikonschicht in einem gemeinsamen Verfahrensschritt, also in einem kombinierten Zustand, ausvulkanisiert. Durch die Vulkanisation erhält das HTV Silikon seine (gewünschten) mechanischen Eigenschaften im (vulkanisierten) Endprodukt. Ferner verbinden sich die beiden im kombinierten Zustand vorliegenden HTV-Silikonschichten während des Vulkanisierens miteinander, sodass sich ein mehrschichtiger HTV Silikonkörper mit einer verlässlich zwischen den HTV-Silikonschichten eingebetteten Sensoreinheit ausbildet. Bevorzugt wird der mehrschichtige HTV Silikonkörper entsprechend der Herstellerangaben des gewählten HTV Silikons in einem Ofen getempert, um den Vulkanisierungsprozess des HTV Silikons zu bewirken. Beispielsweise wird der mehrschichtige HTV Silikonkörper bei 90 °C für zwei Stunden getempert.

In weiterer bevorzugter Ausgestaltung ist vorgesehen, dass das Herstellen der zweiten HTV-Silikonschicht ferner basierend auf einer weiteren Form erfolgt, die einer um eine Schichtdicke der ersten HTV-Silikonschicht angepassten Form der Prothese oder des Überzugs entspricht. Mit anderen Worten ist die weitere Form im Falle einer Negativform um die Schichtdicke der ersten HTV-Silikonschicht reduziert oder im Falle einer Positivform um die Schichtdicke der ersten HTV-Silikonschicht erweitert. Bevorzugt wird die erste HTV-Silikonschicht mit der darauf angeordneten Sensoreinheit und der davon getrennten zweiten HTV-Silikonschicht unter Wärmezufuhr vulkanisiert. Mit anderen Worten werden die erste HTV-Silikonschicht und die zweite HTV-Silikonschicht im voneinander getrennten Zustand ausvulkanisiert. Zur Ausbildung des mehrschichtigen HTV-Silikonkörper werden die dann vulkanisierte erste HTV-Silikonschicht und die vulkanisierte zweite HTV-Silikonschicht bevorzugt unter Verwendung eines Silikonklebers miteinander verbunden. Diese Ausgestaltung hat den Vorteil, dass der Zugriff auf die jeweils zueinander zeigenden Innenseiten der ersten und zweiten HTV-Silikonschichten auch noch im vulkanisierten Zustand möglich ist, beispielsweise um Nacharbeiten vorzunehmen und/oder um weitere Komponenten zwischen der erstem HTV-Silikonschicht und der zweiten HTV-Silikonschicht einbetten zu können, beispielsweise temperaturempfindliche Materialien oder Komponenten, die durch die Wärmezufuhr im Vulkanisierungsschritt beschädigt würden. Die weitere Form ist bevorzugt analog zu der oben genannten Form ausgebildet. Auf eine wiederholende Beschreibung der Merkmale wird daher verzichtet.

In weiterer bevorzugter Ausgestaltung ist vorgesehen, dass eine elektrische Leitung zum Verbinden der Sensoreinheit mit einer Schnittstelle auf der ersten HTV-Silikonschicht angeordnet wird, wobei die elektrische Leitung wenigstens teilweise mäanderartig zwischen der Sensoreinheit und der Schnittstelle verläuft. Eine elektrische Leitung ist ein linienförmiger elektrischer Leiter zum Transport elektrischer Energie oder zur Signalübertragung in der leitungsgebundenen Nachrichten- und Hochfrequenztechnik. Mit anderen Worten dient die elektrische Leitung insbesondere dem Zweck, die von der Sensoreinheit erfassten Messsignale zu einer Auswerte- und/oder Steuereinheit zu leiten, beispielsweise über die oben genannte Schnittstelle. Bevorzugt wird die elektrische Leitung vor dem Herstellen der zweiten HTV-Silikonschicht auf der ersten HTV-Silikonschicht angeordnet. Dadurch, dass die elektrische Leitung wenigstens teilweise mäanderartig verläuft, wird in dem mäanderartigen Bereich eine verbesserte Flexibilität des mehrschichtigen HTV Silikonkörpers erreicht. Bevorzugt weist die elektrische Leitung in den vorhergesehenen Bewegungs- und Knickbereichen der Prothese oder des Überzugs der Prothese einen mäanderartigen Verlauf. Bevorzugt ist die elektrische Leitung zumindest zu 10 Prozent, vorzugsweise zumindest zu 25 Prozent, besonders bevorzugt zumindest zu 50 Prozent, des Abstands zwischen der Sensoreinheit und der Auswerte- oder Steuereinheit oder der Schnittstelle mäanderartig ausgeprägt. Beispielsweise ist die elektrische Leitung als Kupferbahn ausgestaltet. Die vorliegende Offenbarung ist allerdings nicht auf Kupfer beschränkt. Vielmehr können sämtliche elektrisch leitfähige Materialien verwendet werden.

In weiterer bevorzugter Ausgestaltung ist vorgesehen, dass mindestens eine zweite zum Erfassen einer Temperatur und/oder eines Drucks eingerichtete Sensoreinheit auf der zweiten HTV-Silikonschicht (des mehrschichtigen HTV Silikonkörpers) angeordnet wird und eine dritte HTV-Silikonschicht auf der zweiten Sensoreinheit und der zweiten HTV-Silikonschicht basierend auf der HTV Silikon-haltigen Lösung hergestellt wird. Mit anderen Worten wird ein mehrschichtiger HTV Silikonkörper mit einer Vielzahl von Sensoreinheiten zwischen den verschiedenen HTV-Silikonschichten (mindestens 3 HTV-Silikonschichten) aufgebaut werden. Hierdurch wird ein tiefenselektives Erfassen der Temperatur und/des Druckes an der Außenseite der Prothese ermöglicht. Für den beispielhaften Fall, dass nur die weiter außen liegende Sensoreinheit ein Messignal erfasst, die tieferliegende Sensoreinheit hingegen jedoch kein Messignal detektiert, kann die Schlussfolgerung gezogen werden, dass die erfasste Temperatur und/oder der erfasste Druck an der Außenseite der Prothese vergleichsweise gering ist, verglichen zu dem Fall, dass beide Sensoreinheiten Messsignale erfassen. Folglich kann bereits anhand der Existenz beziehungsweise Nichtexistenz von erfassten Messignalen zwischen den verschiedenen Sensoreinheiten eine erste Erkenntnis gezogen werden, ohne die Messignale inhaltlich auszuwerten. Hierdurch kann die Auswertung beziehungsweise Interpretation der Messsignale erleichtert werden. Es ist verständlich, dass die Anzahl an HTV-Silikonschichten und die Anzahl an Sensoreinheiten zwischen den verschiedenen HTV-Silikonschichten vorzugsweise in Abhängigkeit zu einer gewünschten Gesamt-Wandstärke und einer gewünschten Messsensitivität des herzustellenden mehrschichtigen HTV Silikonkörpers gewählt werden können.

In weiterer bevorzugter Ausgestaltung ist vorgesehen, der mehrschichtige HTV Silikonkörper mit einer Gesamtdicke von 0,15 mm bis 1,5 mm hergestellt wird. Mit anderen Worten beträgt die Gesamtwandstärke des mehrschichtigen HTV Silikonkörpers, beispielsweise ein Abstand zwischen den gegenüberliegenden Außenseiten des mehrschichtigen HTV Silikonkörpers, bevorzugt mindestens 0,15 mm, vorzugsweise mindestens 0,25 mm, besonders bevorzugt mindesten 0,5 mm und/oder höchstens 1,5 mm, vorzugsweise höchstens 1,25 mm, besonders bevorzugt höchstens 1 mm. Beispielsweise kann eine Gesamtwandstärke des HTV Silikonkörpers 0,8 mm betragen. Herkömmlich hergestellte Prothesen aus HTV Silikonen benötigen meist eine Oberfläche mit einer "Modulierdicke", also einer Gesamtwandstärke, von mindestens 1,5 mm, 2 mm oder teilweise bis hoch zu 5 mm, um feine Oberflächenstrukturen händisch in die individuelle Prothese einbringen zu können. Die vergleichsweise großen Wandstärken von herkömmlich gefertigten HTV Silikonkörpern haben den Nachteil, dass diese sperrig und unflexibel sind und mitunter Mechaniken der Prothesenhände beeinträchtigen oder sogar kaputt machen können. Zudem ist das Gewicht der Prothese vergleichsweise hoch, sodass der Benutzerkomfort des Patienten verringert ist. Wie bereits erwähnt, gestaltet sich das Erfassen von Messsignalen durch derartig große Wandstärken hindurch ebenfalls als schwierig.

Ein weiterer Aspekt betrifft ein Verfahren zum Herstellen eines mehrschichtigen Silikonliners. Ein (Silikon-)Liner stellt die Verbindung zwischen Stumpf und einer getragenen Prothese dar. Der Liner wird über den Stumpf gezogen oder gerollt, bevor die Prothese angezogen wird. Ein Liner dient grundsätzlich dem Zweck, den Tragekomfort der Prothese zu erhöhen, beispielsweise indem er den Stumpf vor Druckstellen, Hitze und Feuchtigkeit schützt und die Belastungen gleichmäßig verteilt sowie bei hoher Druckbelastung polstert.

Gemäß einem Schritt wird eine Positivform eines Stumpfs eines zu ersetzenden Körperteils bereitgestellt. Die Positivform wird bevorzugt analog zu der Herstellung der Form des oben genannten Verfahrens hergestellt. Auf eine wiederholende Beschreibung der Merkmale wird daher verzichtet.

Ferner wird mindestens eine zum Erzeugen von Temperatur und/oder Druck eingerichtete Aktoreinheit zwischen der Positivform und einer ersten Silikonschicht aus einem ersten Silikon mit einer die Aktoreinheit teilweise freilegenden Öffnung angeordnet. Die erste Silikonschicht ist bevorzugt eine (dünne) Schicht (ausgewalztes) HTV Silikon in einer mittleren Shore-Härte. Eine mittlere Shore-Härte liegt bevorzugt im Bereich von mindestens Shore 20 bis Shore 50, vorzugsweise mindestens Shore 30 bis Shore 40, besonders bevorzugt Shore 35, gemessen nach DIN ISO 48-4. Beispielsweise wird die Aktoreinheit (vorläufig) auf der Positivform fixiert, die erste Silikonschicht auf der Aktoreinheit und zumindest teilweise auf der Positivform angeordnet. Bevorzugt wird die die Aktoreinheit teilweise freilegende Öffnung im Anschluss aus der ersten Silikonschicht ausgeschnitten. In einer bevorzugten alternativen Ausgestaltung ist die Öffnung in der ersten Silikonschicht enthalten, bevor diese auf der Aktoreinheit und zumindest teilweise auf der Positivform angeordnet wird. Dementsprechend muss die erste Silikonschicht derart angeordnet werden, dass die Öffnung die Aktoreinheit zumindest teilweise überlappt. Die Öffnung ist bevorzugt kleiner als die Aktoreinheit. Dies stellt sicher, dass die Aktoreinheit nicht vollständig freiliegt, um diese durch die erste Silikonschicht an der Positivform zu halten. Bevorzugt wird die erste Silikonschicht zumindest einmal um die Positivform herumgewickelt. Dies verbessert die Stabilität des Silikonliners und sorgt für einen passenden Sitz (des Silikonliners) an dem Stumpf.

In einem weiteren Schritt wird eine Leitung durch die Öffnung der ersten Silikonschicht zum Koppeln der Leitung mit der Aktoreinheit geführt. Die Leitung dient dem Zweck, die Aktoreinheit durch die Öffnung hindurch mit einer anderen Komponente verbinden zu können, beispielsweise einer Komponente zur Ansteuerung der Aktoreinheit. Beispiele für die Leitung sind ein Silikonschlauch zum Leiten eines Fluids und eine elektrische Leitung zum Leiten eines elektronischen Ansteuerungssignals. Je nach Ausgestaltung wird die Leitung bevorzugt an der Aktoreinheit fixiert oder mit dieser gekoppelt.

Ferner wird eine zweite Silikonschicht aus einem zweiten Silikon auf einer der Positivform abgewandten Seite der ersten Silikonschicht angeordnet. Das zweite Silikon deckt die Öffnung der ersten Silikonschicht und die Leitung zumindest teilweise ab. Bevorzugt überdeckt das zweite Silikon die Öffnung und die erste Silikonschicht nahezu vollständig. Das zweite Silikon weist ferner eine höhere Shore-Härte als das erste Silikon auf. Bevorzugt ist das zweite Silikon ein (ausgewalztes) HTV Silikon mit einer hohen Shore-Härte. Eine hohe Shore-Härte liegt bevorzugt im Bereich von mindestens Shore 50 bis Shore 100, vorzugsweise mindestens Shore 60 bis Shore 80, besonders bevorzugt Shore 65, gemessen nach DIN ISO 48-4. Durch die höhere Shore-Härte im Vergleich zu der ersten Silikonschicht bietet die zweite Silikonschicht eine bessere Schutzschicht vor äußeren Einflüssen auf den Silikonliner. Bevorzugt bildet die zweite Silikonschicht die äußere Schicht des Silikonliners.

In einem weiteren Verfahrensschritt werden die erste Silikonschicht und die zweite Silikonschicht mit der dazwischen angeordneten Leitung unter Wärmezufuhr vulkanisiert. Mit anderen Worten werden die erste Silikonschicht und die zweite Silikonschicht in einem gemeinsamen Verfahrensschritt, also in einem kombinierten Zustand, ausvulkanisiert. Bevorzugt wird der mehrschichtige Silikonliner entsprechend den Herstellerangaben des gewählten Silikons in einem Ofen getempert, um den Vulkanisierungsprozess des Silikons zu bewirken. Beispielsweise wird der Silikonliner bei 90 °C für zwei Stunden getempert. Durch die Vulkanisation erhält das Silikon seine (gewünschten) mechanischen Eigenschaften im (vulkanisierten) Endprodukt. Ferner verbinden sich die beiden in dem kombinierten Zustand vorliegenden Silikonschichten während des Vulkanisierens miteinander, sodass sich ein mehrschichtiger Silikonliner mit einer verlässlich zwischen den Silikonschichten eingebetteten Aktoreinheit und Leitung ausbildet.

Der erfindungsgemäße Silikonliner ist folglich im auf dem Stumpf getragenen Zustand in der Lage, mittels der Aktoreinheit eine Temperatur und/oder einen Druck auf der Stumpfoberfläche zu erzeugen, welche von dem Träger des Silikonliners wahrnehmbar ist. Es wird vermutet, dass das Auftreten von Phantomschmerzen dadurch verhindert oder zumindest verringert werden kann, dass die fehlende Stimulierung betreffender Gehirnareale aufgrund der ausbleibenden Reizsignale des fehlenden oder eingeschränkten Körperteils, die mutmaßlich zu den Phantomschmerzen führen, durch Stimulation des Stumpfes zum Erzeugen von temperatur- und/oder druckbasierten Reizsignalen ersetzt werden. Durch die Fähigkeit des Silikonliners, eine Temperatur und/oder einen Druck an dem Stumpf des Trägers zu erzeugen, kann demnach den zu Phantomschmerzen führenden komplexen Prozessen im Gehirn des Trägers entgegengewirkt und das Wohlbefinden des Trägers gesteigert werden. Insbesondere in der Kombination mit dem hierin genannten mehrschichtigen HTV Silikonkörpers zur sensorischen Erfassung von Temperaturen und/oder Drücken an der Prothese können die so erfassten Messsignale zur Ansteuerung der Aktoreinheit des Silikonliners herangezogen werden. Dies ermöglicht es dem Träger der Prothese, eine an der Prothese anliegende Temperatur und/oder Druck durch die Aktoreinheit des Silikonliners am Stumpf wahrzunehmen. Nach einer Eingewöhnungszeit ist das Gehirn des Trägers in der Lage, die am Stumpf wirkende Aktoreinheit als Reizsignale der Prothese zu assoziieren. Folglich wird die Funktionalität der Prothese erweitert. Zudem wird die Prothese von dem Träger weniger als Fremdkörper wahrgenommen, sodass die Verwendung des Silikonliners die Akzeptanz des Trägers für die Prothese steigert. Es ist verständlich, dass die Aktoreinheit oder eine Vielzahl von Aktoreinheiten in dem Stumpf entsprechenden Bereichen des Silikonliners (beliebig) verteilt werden können, um dem Träger eine ortsaufgelöste Rückmeldung bezüglich der Temperatur und/oder des Druckes bereitzustellen. Bevorzugt umfasst die Aktoreinheit druckerzeugende Aktoren, wie beispielsweise Piezoaktoren mit mechanischer Übersetzung (z.B. Gebtriebe), volumen-veränderliche Druckbehälter und Druckkissen, und/oder temperaturerzeugende, also kühlende und/oder wärmende, Aktoren, wie Heizdrähte und/oder mit einem Wärmetauscher verbundene Kühl-/Wärmekreisläufe.

In bevorzugter Ausgestaltung ist vorgesehen, dass die Aktoreinheit ein Druckkissen umfasst, welches ein in ein drittes Silikon eingebettetes Stoffgewebe umfasst. Das dritte Silikon weist bevorzugt eine niedrigere Shore-Härte als das erste Silikon auf. Bevorzugt ist das dritte Silikon ein additionsvernetzendes RTV Silikon mit niedriger Shore-Härte. Eine niedrige Shore-Härte liegt bevorzugt im Bereich von mindestens Shore 0020 bis Shore A2, gemessen nach DIN ISO 48-4.

Bevorzugt wird das dritte Silikon auf eine glatte Oberfläche gegossen, das Stoffgewebe hineingelegt und das dritte Silikon über das Stoffgebewege verstrichen. Das Stoffgewebe umfasst bevorzugt ein stretchbares (dehnbares) Verstärkungsgewebe. Das stretchbare Verstärkungsgewebe verbessert die Druckerzeugungseigenschaften des Druckkissens. Nach der Vulkanisierung des dritten Silikons kann das Druckkissen, beispielsweise als rundes oder eckiges Plättchen, aus dem vulkanisierten Produkt ausgeschnitten oder ausgestanzt und zur vorläufigen Fixierung auf der Positivform angeordnet werden. Durch die niedrige Shore-Härte des dritten Silikons ist dieses ausreichend weich, um eine ausblasbare Druckkammer in der Öffnung der ersten Silikonschicht zwischen dem dritten Silikon und der zweiten Silikonschicht auszubilden. Die Druckkammer kann über die Leitung mit Fluiden gefüllt und entleert werden, um den von dem Träger des Silikonliners wahrnehmbaren Druck zu erzeugen.

In weiterer bevorzugter Ausgestaltung ist vorgesehen, dass die Leitung ein Silikonschlauch ist. Der Silikonschlauch umfasst ein Stoffgewebe an dem mit der Aktoreinheit gekoppelten Ende. Das Stoffgewebe ist bevorzugt ein Filzgewebe. Die Verwendung des Stoffgewebes verbessert die Fluidverteilung in das Druckkissen. Bevorzugt wird die Leitung vor dem Einbringen in die Öffnung mit dem Stoffgewebe versehen, wodurch die Herstellung des Silikonliners vereinfacht wird.

In weiterer bevorzugter Ausgestaltung ist vorgesehen, dass die Leitung ein von der ersten Silikonschicht und/oder der zweiten Silikonschicht freistehendes Ende zum Koppeln mit einer Steuereinheit umfasst. Das freistehende Ende ist gegenüberliegend zu dem mit der Aktoreinheit gekoppelten Ende. Mit anderen Worten ragt zumindest ein Ende der Leitung aus dem Silikonliner zwischen der ersten und der zweiten Silikonschicht seitlich hervor. Das freistehende Ende wird bevorzugt mit einer Steuereinheit verbunden. Die Steuereinheit ist bevorzugt zum Ansteuern der Aktoreinheit eingerichtet, beispielsweise mittels elektronischer Steuersignale und/oder mittels Bewegung eines Fluids in der Leitung, beispielsweise zum Erzeugen eines Drucks und/oder zum Transportieren von Wärme (oder Kälte) zu der Aktoreinheit.

In weiterer bevorzugter Ausgestaltung ist vorgesehen, dass vor dem Vulkanisieren der ersten Silikonschicht und der zweiten Silikonschicht mit der dazwischen angeordneten Leitung ein Ende einer Manschette in die erste Silikonschicht und/oder die zweite Silikonschicht eingearbeitet wird. Die Manschette ist bevorzugt ein faserbasierter Überzug, beispielsweise aus Textilien, vorzugsweise aus Naturfasern, wie Baumwolle, und/oder synthetischen Fasern, wie Nylon (chemische Bezeichnung: Polyhexamethylenadipinsäureamid), welcher über das unversehrte Körperteil jenseits des Stumpfes gezogen wird. Dadurch, dass die Manschette vor dem Vulkanisierungsschritt in eine oder beide Silikonschichten eingearbeitet wird, verbindet sich die Manschette während des Vulkanisierungsschritt mit der oder den Silikonschichten. Folglich wird auf einfache Weise eine robuste Verbindung zwischen der Manschette und dem Silikon bereitgestellt. Bevorzugt wird die oben genannte Steuereinheit und/oder andere Komponenten zur Ansteuerung beziehungsweise Aktivierung der Aktoreinheit auf der Manschette angeordnet.

Beispiele für andere Komponenten sind Kommunikationseinheiten zur drahtlosen und/oder drahtgebundenen Kommunikation mit der Prothese (zum Empfangen von Messignalen der Sensoreinheit/en), Datenverarbeitungseinheiten, wie Mikrocontroller, und Batteriespeicher zum Bereitstellen der erforderlichen elektrischen Energie. Bevorzugt erfolgt eine Kommunikation zwischen der Prothese und dem Silikonliner drahtlos, beispielsweise mittels Austausch von Bluetoothsignalen. Mit anderen Worten umfasst die Prothese bevorzugt eine zwischen der ersten und zweiten HTV-Silikonschicht eingebettete Kommunikationseinheit, die dazu eingerichtet ist, Kommunikationssignale, insbesondere die Messsignale der Sensoreinheit/en, an den Silikonliner zu übertragen. Der Silikonliner umfasst bevorzugt eine Kommunikationseinheit auf der Manschette, welche dazu eingerichtet ist, Kommunikationssignale, insbesondere die Messsignale der Sensoreinheit/en, von der Prothese zu empfangen. Die empfangenen Kommunikationssignale werden bevorzugt zu der Steuereinheit auf der Manschette zur Ansteuerung beziehungsweise Aktivierung der Aktoreinheit/en des Silikonliners gemäß den Messsignalen der Sensoreinheit/en der Prothese weitergeleitet. Demnach kann der Träger der Prothese eine an der Prothese herrschende Temperatur und/oder einen an der Prothese herrschenden Druck unter Verwendung der Aktoreinheit/en wahrnehmen. Folglich wird die Akzeptanz und die Funktionalität der Prothese verbessert.

Ein weiterer Aspekt betrifft einen mehrschichtigen HTV Silikonkörper für eine Prothese oder einen Überzug der Prothese. Der mehrschichtige HTV Silikonkörper ist durch das oben genannte Verfahren zum Herstellen eines mehrschichtigen HTV Silikonkörpers für eine Prothese oder einen Überzug der Prothese herstellbar. Die mit den Verfahren beschriebenen (optionalen) Merkmale und deren Vorteile werden analog in dem mehrschichtigen HTV Silikonkörper verwirklicht und sind daher beliebig miteinander kombinierbar.

Ein weiterer Aspekt betrifft einen mehrschichtigen Silikonliner (zum Tragen unter einer Prothese oder eines Überzug einer Prothese). Der mehrschichtige Silikonliner ist durch das oben genannte Verfahren zum Herstellen eines mehrschichtigen Silikonliners herstellbar. Die mit den Verfahren beschriebenen (optionalen) Merkmale und deren Vorteile werden analog in dem mehrschichtigen Silikonliner verwirklicht und sind daher beliebig miteinander kombinierbar.

Ein weiterer Aspekt betrifft ein System zur sensorischen Rückmeldung an einen Träger einer Prothese oder eines Überzugs der Prothese. Das System umfasst den obigen mehrschichtigen HTV Silikonkörper oder eine Prothese mit einem Silikonüberzug und einer zwischen der Prothese und dem Silikonüberzug angeordneten zum Erfassen einer Temperatur und/oder eines Drucks eingerichteten Sensoreinheit und den obigen mehrschichtigen Silikonliner. Der mehrschichtige HTV Silikonkörper oder die Prothese und der mehrschichtige Silikonliner sind dazu eingerichtet, derart in einem Datenaustausch zu stehen, dass eine durch die Sensoreinheit des mehrschichtigen HTV Silikonkörpers oder der Prothese erfasste Temperatur und/oder ein durch die Sensoreinheit des mehrschichtigen HTV Silikonkörpers oder der Prothese erfasster Druck als Kommunikationssignal zum Antreiben der Aktoreinheit des mehrschichtigen Silikonliners an diesen übermittelt wird. Die mit dem mehrschichtigen HTV Silikonkörper und dem mehrschichtigen Silikonliner beschriebenen (optionalen) Merkmale und deren Vorteile werden analog in dem System verwirklicht und sind daher beliebig miteinander kombinierbar. Die Prothese mit dem Silikonüberzug umfasst bevorzugt eine mechanische Grundstruktur, die zumindest eine ursprüngliche Funktion der fehlenden Gliedmaße wiederherstellt, beispielsweise eine Mechanik, die zumindest eine Bewegungsfunktion der fehlenden Gliedmaße bereitstellt. Bevorzugt ist über die mechanische Grundstruktur eine Zwischenschicht gezogen, beispielsweise ein Textilstoff. In die Zwischenschicht beziehungsweise auf der Außenseite der Zwischenschicht wird bevorzugt die zum Erfassen einer Temperatur und/oder eines Drucks eingerichtete Sensoreinheit eingebettet beziehungsweise angeordnet. Der Silikonüberzug ist bevorzugt über beziehungsweise auf der Zwischenschicht angeordnet. Ferner bevorzugt ist der Silikonüberzug analog zu der ersten HTV-Silikonschicht gemäß dem obigen Verfahren hergestellt, also basierend auf einer HTV Silikon-haltigen Lösung und einer Form des Überzugs. Vorteilhaft kann der Silikonüberzug somit besonders dünn, beispielsweise zwischen 0,15 mm bis 1,5 mm, und lebensecht hergestellt werden.

Weitere bevorzugte Ausgestaltungen der Erfindung ergeben sich aus den übrigen, in den Unteransprüchen genannten Merkmalen.

Die verschiedenen in dieser Anmeldung genannten Ausführungsformen der Erfindung sind, sofern im Einzelfall nicht anders ausgeführt, mit Vorteil miteinander kombinierbar.

Die Erfindung wird nachfolgend in Ausführungsbeispielen anhand der zugehörigen Zeichnungen erläutert. Es zeigen:
- Figur 1: eine schematische Darstellung eines Systems zur sensorischen Rückmeldung gemäß einer Ausführungsform;
- Figur 2: eine schematische Darstellung eines hergestellten mehrschichtigen HTV Silikonkörpers gemäß einer Ausführungsform;
- Figur 3: eine schematische Querschnittsdarstellung des hergestellten mehrschichtigen HTV Silikonkörpers gemäß einer Ausführungsform;
- Figuren 4a-c: schematische Darstellungen von einzelnen Herstellungsschritten eines mehrschichtigen Silikonliners gemäß einer Ausführungsform;
- Figur 5: eine schematische Querschnitts-Darstellung eines mehrschichtigen Silikonliners gemäß einer Ausführungsform;
- Figur 6: eine schematische Darstellung eines Verfahren zum Herstellen eines mehrschichtigen HTV Silikonkörpers gemäß einer Durchführungsform; und
- Figur 7: eine schematische Darstellung eines Verfahrens zum Herstellen eines mehrschichtigen Silikonliners gemäß einer Durchführungsform.

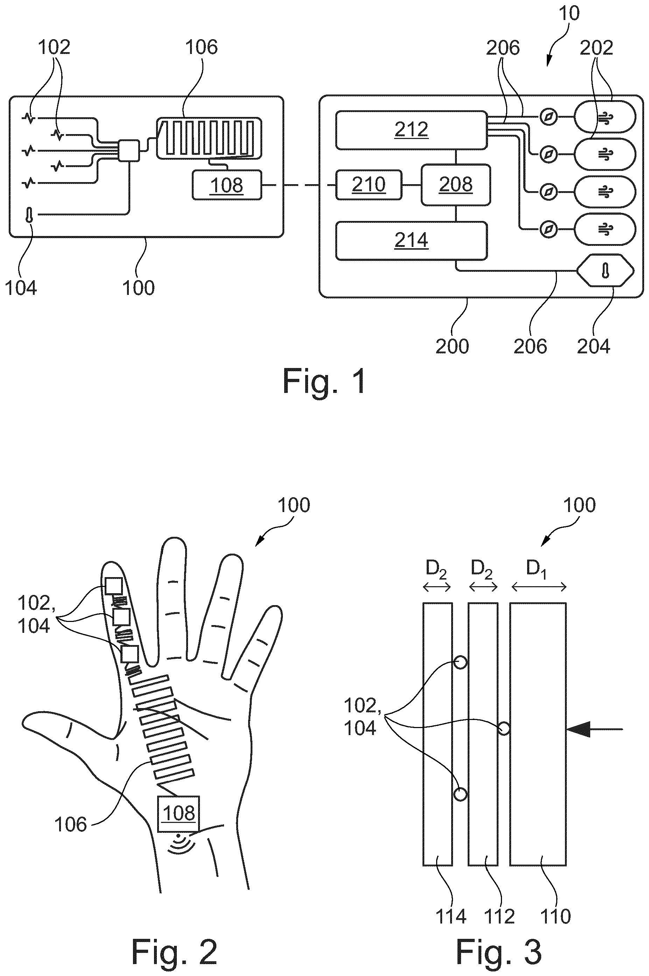

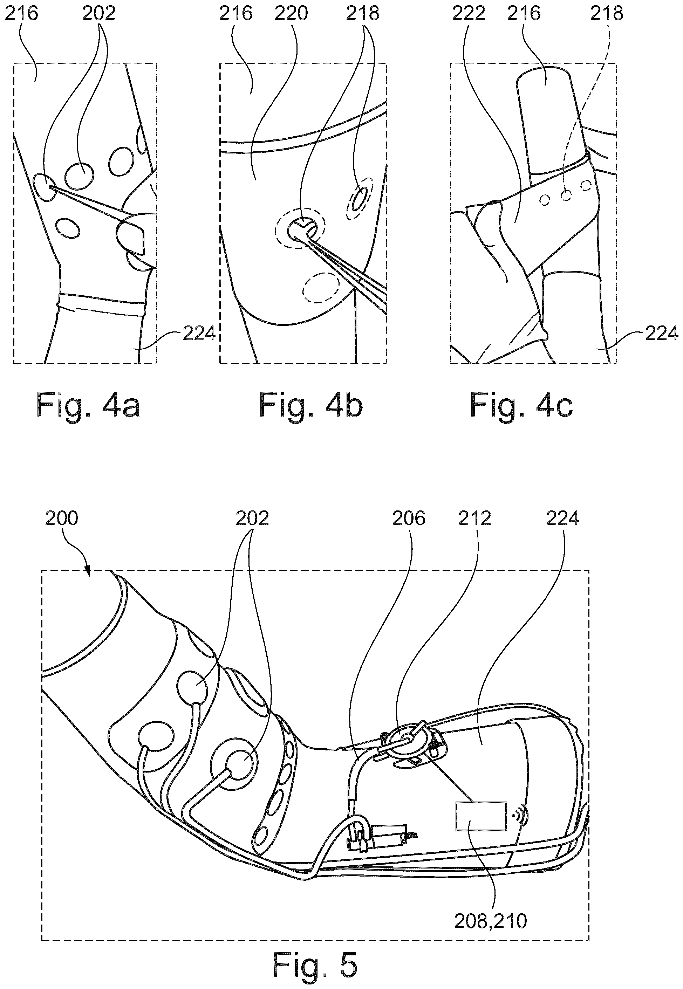

Figur 1 eine schematische Darstellung eines Systems 10 zur sensorischen Rückmeldung an einen Träger einer Prothese oder eines Überzugs der Prothese gemäß einer Ausführungsform. Das System 10 umfasst einen mehrschichtigen HTV Silikonkörper 100 und einen mehrschichtigen Silikonliner 200, die jeweils schematisch in der Figur 1 abgebildet sind. Mögliche Ausgestaltungsformen des mehrschichtigen Silikonkörpers 100 werden im Hinblick auf die Figuren 2 und 3 näher beschrieben. Ausgestaltungen des mehrschichtigen Silikonliners 200 werden in den Figuren 4a-c und 5 näher erläutert.

Der mehrschichtige HTV Silikonkörper 100 wird als eine Prothese oder als ein Überzug für eine Prothese verwendet, welche es ermöglicht, an der Prothese auftretende Temperaturen und/oder oder auf die Prothese einwirkende Drücke sensorisch zu erfassen. Der mehrschichtige HTV Silikonkörper 100 umfasst eine Vielzahl von zum Erfassen einer Temperatur und/oder eines Drucks eingerichtete Sensoreinheiten 102, 104, eine elektrische Leitung 106 und eine Schnittstelle 108.

Die Sensoreinheiten 102, 104 sind jeweils dazu eingerichtet, die Temperatur und/oder den Druck an der Außenseite des HTV Silikonkörpers 100, also an der Außenseite der Prothese oder des Überzugs, zu erfassen. Die Sensoreinheiten 102, 104 sind jeweils mindestens zwischen einer ersten HTV-Silikonschicht 110 und einer zweiten HTV-Silikonschicht 112 eingebettet, wie es im Hinblick auf Figur 3 näher beschrieben wird.

In der beispielhaften in Figur 1 gezeigten Ausgestaltung umfasst der HTV Silikonkörper 100 vier zum Erfassen eines Druckes eingerichtete Sensoreinheiten 102 und eine zum Erfassen einer Temperatur eingerichtete Sensoreinheit 104. Da sich die Temperatur im Vergleich zum Druck ungedämpfter über das Material einer Prothese verteilen kann, ist die Anzahl an zum Erfassen der Temperatur eingerichteten Sensoreinheiten 104 bevorzugt geringer als die Anzahl an zum Erfassen des Druckes eingerichtete Sensoreinheiten 102. Die dargestellte Anzahl an Sensoreinheiten 102, 104 ist allerdings lediglich exemplarischer Natur und nicht darauf beschränkt. Vielmehr kann eine beliebige Anzahl an Sensoreinheiten 102, 104 in einem beliebigen Verhältnis zueinander vorgesehen sein. Es ist verständlich, dass die Anzahl und das Verhältnis der Sensoreinheiten 102, 104 von der verwendeten Prothese, ihrer Größe und der gewünschten sensorischen Fähigkeit abhängig gemacht werden kann. Die zum Erfassen des Druckes eingerichteten Sensoreinheiten 102 sind beispielsweise druckabhängige elektrische Widerstände oder auf dem Piezoeffekt-basierte Bauteile, ohne hierauf beschränkt zu sein. Die zum Erfassen der Temperatur eingerichtete Sensoreinheit 104 ist beispielsweise ein temperaturabhängiger elektrischer Widerstand oder ein auf dem Seebeck-Effekt-basiertes Bauteil, ohne darauf beschränkt zu sein.

Die Sensoreinheiten 102, 104 des HTV Silikonkörpers 100 sind mit einem optionalen Mikrocontroller (kein Bezugszeichen) verbunden, der die erfassten Messignale der Sensoreinheiten 102, 104 empfängt und über die elektrische Leitung 106 an die Schnittstelle 108 übermittelt. Der Mikrocontroller und die elektrische Leitung 106 sind ebenfalls zwischen der ersten HTV-Silikonschicht 110 und der zweiten HTV-Silikonschicht 112 eingebettet. Demnach sind die Sensoreinheiten 102, 104, die elektrische Leitung 106 und der Mikrocontroller nach außen durch die HTV-Silikonschichten 110, 112 verdeckt und vor äußeren Einflüssen geschützt.

Figur 2 zeigt eine schematische Darstellung eines hergestellten mehrschichtigen HTV Silikonkörpers 100 gemäß einer Ausführungsform. In der Figur 2 gezeigten Ausgestaltung ist der mehrschichtige HTV Silikonkörper 100 als Handprothese dargestellt. Jedoch ist die vorliegende Offenbarung nicht darauf beschränkt. Es ist verständlich, dass der hierin offenbarte mehrschichtige HTV Silikonkörper 100 als Prothese oder Überzug für eine Prothese zum Ersatz eines fehlenden oder eingeschränkten Körperteils dient. Betroffene Körperteile sind beispielsweise Finger, eine Hand, ein Arm, ein Bein, ein Fuß und dergleichen oder auch nur ein Teile davon, beispielsweise eine Teilhand oder ein Teilfuß.

Die dargestellte Handprothese zeigt drei Sensoreinheiten 102, 104 an dem Zeigefinger der inneren Handseite. Es ist verständlich, dass analog Sensoreinheiten 102, 104 an mehreren oder allen Fingern und/oder an der Handfläche und/oder an der äußeren Handseite angeordnet sein können. Im Wesentlich können die Sensoreinheiten 102, 104 überall dort angeordnet sein, wo eine Messung der Temperatur und/oder des Druckes an der Prothese sinnvoll erscheint, beispielsweise weil diese häufig in Berührung mit anderen Gegenständen gelangen. Die Sensoreinheiten 102, 104 sind über zumindest einen elektrischen Leiter 106 mit der Schnittstelle 108 verbunden. Die Schnittstelle 108 ist beispielweise im Handwurzelbereich angeordnet.

Figur 2 zeigt ferner, dass die elektrische Leitung 106 wenigstens teilweise mäanderartig zwischen den Sensoreinheiten 102, 104 und der Schnittstelle 108 verläuft. Dadurch, dass die elektrische Leitung 106 wenigstens teilweise mäanderartig verläuft, wird in den mäanderartigen Bereichen im Gegensatz zu einem (ausschließlich) geraden Verlauf eine verbesserte Flexibilität des mehrschichtigen HTV Silikonkörpers 100 erreicht. Insofern weist die elektrische Leitung 106 in den vorhergesehenen Bewegungs- und Knickbereichen der Prothese oder des Überzugs der Prothese, wie den Fingergelenken und der Handinnenfläche, einen mäanderartigen Verlauf. Beispielsweise ist die elektrische Leitung 106 als Kupferbahn ausgestaltet.

Figur 3 zeigt eine schematische Querschnittsdarstellung des hergestellten mehrschichtigen HTV Silikonkörpers 100 gemäß einer Ausführungsform. In der Querschnittsdarstellung ist die Einbettung der Sensoreinheiten 102, 104 zwischen beispielhaften drei HTV-Silikonschichten 110, 112, 114 dargestellt.

Die erste HTV-Silikonschicht 110 wurde basierend auf einer HTV Silikon-haltigen Lösung und einer Form der Prothese oder des Überzugs der Prothese hergestellt. Das HTV Silikon der HTV Silikon-haltigen Lösung lag in einem Lösungsmittel gelöst vor. Durch die Verwendung der HTV Silikon-haltigen Lösung können HTV Silikonschichten 110, 112, 114 mit erheblich geringeren Schichtdicken D₁, D₂ (Wandstärken) hergestellt werden als bei der konventionellen Festkörper-Verarbeitung von HTV Silikonen. Ferner können unterschiedliche HTV Silikon-haltige Lösungen zur Ausbildung von HTV-Silikonschichten 110, 112, 114 unterschiedlicher Schichtdicke D₁, D₂ hergestellt werden. In der beispielhaft in Figur 3 gezeigten Ausgestaltung des HTV Silikonkörpers 100 ist die erste HTV-Silikonschicht 110 mit einer größeren ersten Schichtdicke D₁ ausgeprägt als die zweite und dritte HTV-Silikonschichten 112, 114. Hierdurch bietet die erste HTV-Silikonschicht 110 eine erhöhte Widerstandskraft. Da die erste HTV-Silikonschicht 110 als äußere Schicht der Prothese fungiert, wird somit die Lebensdauer der Prothese erhöht und gleichzeitig eine ausreichende Flexibilität durch die dünneren innenliegenden HTV-Silikonschichten 112, 114 erreicht. Zur Verdeutlichung von auf die Prothese wirkende äußere Einflüsse, wie Druck oder Temperatur, ist ein auf die erste HTV-Silikonschicht 110 gerichteter Pfeil eingezeichnet. Die ersten Schichtdicke D₁ beträgt beispielsweise 0,4 mm, während die zweite Schichtdicke D₂ beispielsweise 0,2 mm beträgt. Folglich beträgt die Gesamtwandstärke des mehrschichtigen HTV Silikonkörpers 100 im Wesentlichen 0,8 mm.

Nach dem Ausformen der ersten HTV-Silikonschicht 110 mit Hilfe der Form wird eine erste Vielzahl von Sensoreinheiten 102, 104 auf der ersten HTV-Silikonschicht 110 angeordnet. Anschließend wird die zweite HTV-Silikonschicht 112 auf die erste HTV-Silikonschicht 110 und die darauf angeordnete erste Vielzahl von Sensoreinheiten 102, 104 hergestellt. Ferner wird eine zweite Vielzahl von Sensoreinheiten 102, 104 auf der zweiten HTV-Silikonschicht 112 angeordnet und eine dritte HTV-Silikonschicht 114 auf der zweiten Vielzahl von Sensoreinheiten 102, 104 und der zweiten HTV-Silikonschicht 112 hergestellt. Durch die anschließende Vulkanisation der HTV-Silikonschichten 110, 112, 114werden die Sensoreinheiten 102, 104 verlässlich in dem HTV Silikonkörper 100 eingebettet, wie es in Figur 3 dargestellt ist.

Im Nachfolgenden wird der mehrschichtige Silikonliner 200 im Hinblick auf die Figuren 1, 4a-c und 5 näher beschrieben.

Der mehrschichtige Silikonliner 200 umfasst eine Vielzahl von zum Erzeugen von Temperatur und/oder Druck eingerichtete Aktoreinheiten 202, 204, eine Vielzahl von Leitungen 206, eine über die Leitungen 206 mit den Aktoreinheiten 202, 204 verbundene Steuereinheit 208, eine mit der Steuereinheit 208 verbundene Schnittstelle 210 sowie eine Druckerzeugungseinheit 212 und eine Temperaturerzeugungseinheit 214.

Die Aktoreinheiten 202, 204 umfassen eine Vielzahl von zum Erzeugen von Druck eingerichtete Aktoreinheiten 202, beispielsweise Druckkissen, und eine Vielzahl von zum Erzeugen von Temperatur eingerichteten Aktoreinheiten 204, beispielsweise Heizdrähte. Die Leitungen 206 der zum Erzeugen von Druck eingerichteten Aktoreinheiten 202 sind jeweils dazu eingerichtet, ein Fluid zwischen der Druckerzeugungseinheit 212 und der jeweiligen Aktoreinheit 202 hin und her zu befördern. Die Leitungen 206 der zum Erzeugen von Temperatur eingerichteten Aktoreinheiten 204 sind jeweils dazu eingerichtet, einen elektrischen Strom von der Temperaturerzeugungseinheit 214 und der jeweiligen Aktoreinheit 204 hin und zurück zu befördern. Die Steuereinheit 208 ist dazu eingerichtet, die Druckerzeugungseinheit 212 und die Temperaturerzeugungseinheit 214 zum Aktivieren der jeweiligen Aktoreinheiten 202, 204 anzusteuern, beispielsweise mittels elektronischer Ansteuerungssignale.

Die Druckerzeugungseinheit 212, beispielsweise ein Pumpenmodul, ist jeweils über eine Leitung 206 mit vier zum Erzeugen von Druck eingerichteten Aktoreinheiten 202 und über eine elektrische Leitung mit der Steuereinheit 208 verbunden. Die Temperaturerzeugungseinheit 214, beispielsweise ein ansteuerbarer Stromwandler zum Aktivieren des Heizdrahtes, ist über eine Leitung 206 mit einer zum Erzeugen von Temperatur eingerichteten Aktoreinheit 204 und über eine weitere elektrische Leitung mit der Steuereinheit 208 verbunden. Die dargestellte Anzahl an Aktoreinheiten 202, 204 ist allerdings lediglich exemplarischer Natur und nicht darauf beschränkt. Vielmehr kann eine beliebige Anzahl an Aktoreinheiten 202, 204 in einem beliebigen Verhältnis zueinander vorgesehen sein. Es ist verständlich, dass die Anzahl und das Verhältnis der Aktoreinheiten 202, 204 von dem verwendeten mehrschichtigen Silikonliner 200, seiner Größe und den gewünschten aktorischen Fähigkeit abhängig gemacht werden kann. Bevorzugt entspricht die Anzahl an Aktoreinheiten 202, 204 der Anzahl an Sensoreinheiten 102, 104 des oben beschriebenen HTV Silikonkörpers, also der verwendeten Prothese. Insofern kann jeweils eine Aktoreinheit 202, 204 gemäß den Messignalen einer Sensoreinheit 102, 104 angesteuert werden.

Der mehrschichtige HTV Silikonkörper 100 und der mehrschichtige Silikonliner 200 sind dazu eingerichtet, derart in einem Datenaustausch zu stehen, dass eine durch die Sensoreinheiten 102, 104 des mehrschichtigen HTV Silikonkörpers 100 erfasste Temperaturen und Drücke an den mehrschichtigen Silikonliner 200 zu übermitteln. Dies erfolgt unter Verwendung der beiden Schnittstellen 108 und 210. Die Schnittstelle 108 umfasst eine Kommunikationseinheit, die die dazu eingerichtet ist, Kommunikationssignale, nämlich die erfassten Messsignale der Sensoreinheiten 102, 104, an den mehrschichtigen Silikonliner 200 zu übertragen. Die Schnittstelle 210 des mehrschichtigen Silikonliners 200 umfasst ebenfalls eine Kommunikationseinheit, die dazu eingerichtet ist, Kommunikationssignale, nämlich die erfassten Messsignale der Sensoreinheiten 102, 104, von der Prothese zu empfangen. Die empfangenen Kommunikationssignale werden zu der Steuereinheit 208 weitergeleitet und zur Ansteuerung der Aktoreinheiten 202, 204 des mehrschichtigen Silikonliners 200 verwendet. Demnach kann der Träger der Prothese an der Prothese herrschende Temperaturen und an der Prothese herrschende Drücke unter Verwendung der Aktoreinheiten 202, 204 wahrnehmen. Folglich wird die Akzeptanz und die Funktionalität der Prothese verbessert.

Figuren 4a-c zeigen schematische Darstellungen von einzelnen Herstellungsschritten eines mehrschichtigen Silikonliners 200 gemäß einer Ausführungsform. Auf eine bereitgestellte Positivform 216 eines beispielhaften Armstumpfs werden als Druckkissen ausgestaltete Aktoreinheiten 202 vorläufig fixiert (siehe Figur 4a). Eine erste Silikonschicht 220 in Gestalt einer Schicht von ausgewalztem HTV Silikon einer mittleren Shore-Härte zwischen Shore 20 bis Shore 50 wird auf die Druckkissen gelegt und um die Positivform 216 herumgewickelt. Dann wird je eine Öffnung 218 in die erste Silikonschicht 220 in die die Aktoreinheiten 202 überlagernden Bereiche eingebracht (siehe Figur 4b). Dann wird jeweils eine Leitung 206 durch die Öffnungen 218 der ersten Silikonschicht 220 zum Koppeln mit den jeweiligen Aktoreinheit 202 geführt. Die Leitungen 206 dienen dem Zweck, die Aktoreinheiten 202 durch die Öffnungen 218 hindurch mit der Druckerzeugungseinheit 212 verbinden zu können.

Anschließend wird eine zweite Silikonschicht 222 aus einem zweiten Silikon auf einer der Positivform 216 abgewandten Seite der ersten Silikonschicht 220 angeordnet (siehe Figur 4c). Die zweite Silikonschicht deckt die Öffnungen 218 und die erste Silikonschicht 220 im Wesentlichen vollständig und die Leitungen 206 zumindest teilweise ab. Das zweite Silikon ist ein ausgewalztes HTV Silikon mit einer hohen Shore-Härte von mindestens Shore 50 bis Shore 100. Durch die höhere Shore-Härte im Vergleich zu der ersten Silikonschicht 220 bietet die zweite Silikonschicht 222 eine bessere Schutzschicht vor äußeren Einflüssen auf den mehrschichtigen Silikonliner 200. Demnach bildet die zweite Silikonschicht 220 die äußere Schicht des mehrschichtigen Silikonliners 200. Vor dem Vulkanisieren der Silikone des erhaltenen mehrschichtigen Silikonkörpers 200 wird eine Manschette 224 zwischen die erste Silikonschicht 220 und die zweite Silikonschicht 220 eingearbeitet. In dem Vulkanisierungsschritt verbindet sich die Manschette 224 dann mit den Silikonschichten 220, 222. Folglich wird auf einfache Weise eine robuste Verbindung zwischen der Manschette 224 und den Silikonschichten 220, 222 bereitgestellt.

Figur 5 zeigt eine schematische Querschnitts-Darstellung eines mehrschichtigen Silikonliners 200 im vulkanisierten Zustand gemäß einer Ausführungsform. Auf der Manschette 224 sind weitere Komponenten, wie die Steuereinheit 208, die die oben beschriebene Kommunikationseinheit umfassende Schnittstelle 210 und die Druckerzeugungseinheit 212, angeordnet. Die Druckerzeugungseinheit 212 ist einem zwischen der ersten Silikonschicht 220 und der zweiten Silikonschicht 222 herausragenden Enden der Leitungen 206 verbunden. Ein von der Schnittstelle 108 des HTV Silikonkörpers 100 ausgesendetes und von der Kommunikationseinheit der Schnittstelle 210 des Silikonliners 200 empfangenes Kommunikationssignal wird an die Steuereinheit 208 übergeben und zu einem Ansteuerungssignal für die Druckerzeugungseinheit 212 umgewandelt. Die Druckerzeugungseinheit 212 erzeugt in den Leitungen 206 einen Druck, welcher in den als Druckkissen ausgestalteten Aktoreinheiten 202 gelangt. Die Druckkissen werden durch den Druck ausgedehnt. Da die zweite Silikonschicht eine hohe Shore-Härte aufweist, bläst sich das Druckkissen im Wesentlichen in Richtung des Armstumpfs aus. Dort wird es von dem Träger des mehrschichtigen Silikonliners 200 wahrgenommen. Es ist verständlich, dass der in den Figuren 4a-c und 5 offenbarte mehrschichtige Silikonliner 200 nicht auf die die reine Druckerzeugungsfunktion beschränkt ist. Vielmehr kann der mehrschichtige Silikonliner 200 zusätzlich oder alternativ eine analoge Temperaturerzeugung aufweisen.

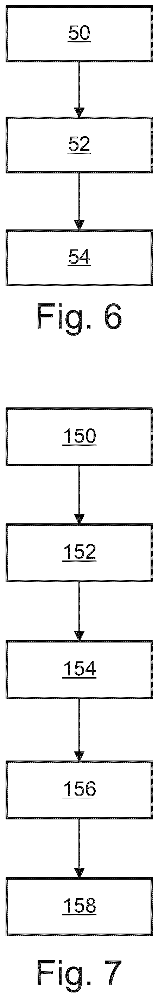

Figur 6 zeigt eine schematische Darstellung eines Verfahren zum Herstellen eines mehrschichtigen HTV Silikonkörpers 100 gemäß einer Durchführungsform.

In einem ersten Verfahrensschritt 50 wird eine erste HTV-Silikonschicht 110 basierend auf einer HTV Silikon-haltigen Lösung und einer Form der Prothese oder des Überzugs hergestellt. Das HTV Silikon der HTV Silikon-haltigen Lösung ist hierbei in einem Lösungsmittel gelöst.

Gemäß einem zweiten Verfahrensschritt 52 wird mindestens eine zum Erfassen einer Temperatur und/oder eines Drucks eingerichteten Sensoreinheit 102, 104 auf der ersten HTV-Silikonschicht 110 angeordnet.

In einem dritten Verfahrensschritt 54 wird eine zweiten HTV-Silikonschicht 112 auf der Sensoreinheit 102, 104 und der ersten HTV-Silikonschicht 110 basierend auf der (oder einer anderen) HTV Silikon-haltigen Lösung hergestellt.

Figur 7 zeigt eine schematische Darstellung eines Verfahrens zum Herstellen eines mehrschichtigen Silikonliners 200 gemäß einer Durchführungsform.

In einem ersten Verfahrensschritt 150 wird eine Positivform 216 eines Stumpfs eines zu ersetzenden Körperteils bereitgestellt.

Gemäß einem zweiten Verfahrensschritt 152 wird mindestens eine zum Erzeugen von Temperatur und/oder Druck eingerichtete Aktoreinheit 202, 204 zwischen der Positivform 216 und einer ersten Silikonschicht 220 aus einem ersten Silikon mit einer die Aktoreinheit 202, 204 teilweise freilegenden Öffnung 218 angeordnet (siehe Figuren 4a+b).

In einem dritten Verfahrensschritt 154 wird eine Leitung 206 durch die Öffnung 218 der ersten Silikonschicht 220 zum Koppeln der Leitung 206 mit der Aktoreinheit 202, 204 geführt.

Gemäß einem vierten Verfahrensschritt 156 wird eine zweiten Silikonschicht 222 aus einem zweiten Silikon auf einer der Positivform 216 abgewandten Seite der ersten Silikonschicht 220 angeordnet (siehe Figur 4c). Das zweite Silikon deckt die Öffnung 218 der ersten Silikonschicht 220 und die Leitung 206 zumindest teilweise ab und weist eine höhere Shore-Härte als das erste Silikon auf.

In einem fünften Verfahrensschritt 158 werden die erste Silikonschicht 220 und die zweite Silikonschicht 222 mit der dazwischen angeordneten Leitung 206 unter Wärmezufuhr vulkanisiert.

### Bezugszeichenliste

- 10: System zur sensorischen Rückmeldung

- 100: mehrschichtiger HTV Silikonkörper
- 102: Sensoreinheit (Druck)
- 104: Sensoreinheit (Temperatur)
- 106: elektrische Leitung
- 108: Schnittstelle
- 110: erste HTV-Silikonschicht
- 112: zweite HTV-Silikonschicht
- 114: dritte HTV-Silikonschicht

- 200: mehrschichtiger Silikonliner
- 202: Aktoreinheit (Druck)
- 204: Aktoreinheit (Temperatur)
- 206: Leitung
- 208: Steuereinheit
- 210: Schnittstelle
- 212: Druckerzeugungseinheit
- 214: Temperaturerzeugungseinheit
- 216: Positivform eines Stumpfs
- 218: Öffnung
- 220: erste Silikonschicht
- 222: zweite Silikonschicht
- 224: Manschette

- D₁: erste Schichtdicke
- D₂: zweite Schichtdicke

- 50: erster Verfahrensschritt - Herstellen einer ersten HTV-Silikonschicht
- 52: zweiter Verfahrensschritt - Anordnen einer Sensoreinheit
- 54: dritter Verfahrensschritt - Herstellen einer zweiten HTV-Silikonschicht

- 150: erster Verfahrensschritt - Bereitstellen einer Positivform

- 152: zweiter Verfahrensschritt - Anordnen einer Aktoreinheit zwischen einer ersten Silikonschicht und der Positivform
- 154: dritter Verfahrensschritt - Führen einer Leitung durch eine Öffnung
- 156: vierter Verfahrensschritt - Anordnen einer zweiten Silikonschicht
- 158: fünfter Verfahrensschritt - Vulkanisieren der Silikonschichten

## Patentansprüche

1. Verfahren zum Herstellen eines mehrschichtigen HTV Silikonkörpers (100) für eine Prothese oder einen Überzug der Prothese, wobei das Verfahren die Schritte umfasst:
- Herstellen (50) einer ersten HTV-Silikonschicht (110) basierend auf einer HTV Silikon-haltigen Lösung und einer Form der Prothese oder des Überzugs, wobei das HTV Silikon der HTV Silikon-haltigen Lösung in einem Lösungsmittel gelöst ist,
- Anordnen (52) mindestens einer zum Erfassen einer Temperatur und/oder eines Drucks eingerichteten Sensoreinheit (102, 104) auf der ersten HTV-Silikonschicht (110), und
- Herstellen (54) einer zweiten HTV-Silikonschicht (112) auf der Sensoreinheit (102, 104) und der ersten HTV-Silikonschicht (110) basierend auf der HTV Silikon-haltigen Lösung.

2. Verfahren nach Anspruch 1, wobei die Form der Prothese oder des Überzugs eine Negativ- und/oder Positivform der Prothese oder des Überzugs umfasst und das Herstellen der ersten HTV-Silikonschicht (110) einen der folgenden Schritte umfasst:
- Ausschwenken der HTV Silikon-haltigen Lösung in der Negativform oder
- Übergießen der Positivform mit der HTV Silikon-haltigen Lösung und/oder Eintauchen der Positivform in die HTV Silikon-haltige Lösung.

3. Verfahren nach einem der vorangehenden Ansprüche, welches ferner den Schritt umfasst:
- Vulkanisieren der ersten HTV-Silikonschicht (110) und der darauf angeordneten zweiten HTV-Silikonschicht (112) mit der dazwischen angeordneten Sensoreinheit (102, 104) unter Wärmezufuhr.

4. Verfahren nach einem der Ansprüche 1 oder 2, wobei das Herstellen (54) der zweiten HTV-Silikonschicht (112) ferner basierend auf einer weiteren Form erfolgt, die einer um eine Schichtdicke der ersten HTV-Silikonschicht (110) angepassten Form der Prothese oder des Überzugs entspricht, und wobei das Verfahren ferner die Schritte umfasst:
- Vulkanisieren der ersten HTV-Silikonschicht (110) mit der darauf angeordneten Sensoreinheit (102, 104) und der davon getrennten zweiten HTV-Silikonschicht (112) unter Wärmezufuhr und
- Verbinden der vulkanisierten ersten HTV-Silikonschicht (110) und der vulkanisierten zweiten HTV-Silikonschicht (112) unter Verwendung eines Silikonklebers.

5. Verfahren nach einem der vorangehenden Ansprüche, wobei eine elektrische Leitung (106) zum Verbinden der Sensoreinheit (102, 104) mit einer Schnittstelle (108) auf die erste HTV-Silikonschicht (110) angeordnet wird, wobei die elektrische Leitung (106) wenigstens teilweise mäanderartig zwischen der Sensoreinheit (102, 104) und der Schnittstelle (108) verläuft.

6. Verfahren nach einem der vorangehenden Ansprüche, ferner den Schritt umfassend:
- Anordnen mindestens einer zweiten zum Erfassen einer Temperatur und/oder eines Drucks eingerichteten Sensoreinheit (102, 104) auf der zweiten HTV-Silikonschicht (112), und
- Herstellen einer dritten HTV-Silikonschicht (114) auf der zweiten Sensoreinheit (102, 104) und der zweiten HTV-Silikonschicht (112) basierend auf der HTV Silikon-haltigen Lösung.

7. Verfahren nach einem der vorangehenden Ansprüche, wobei der mehrschichtige HTV Silikonkörper (100) mit einer Gesamtdicke von 0,15 mm bis 1,5 mm hergestellt wird.

8. Verfahren zum Herstellen eines mehrschichtigen Silikonliners (200), wobei das Verfahren die folgenden Schritte aufweist:
- Bereitstellen (150) einer Positivform (216) eines Stumpfs eines zu ersetzenden Körperteils,
- Anordnen (152) mindestens einer zum Erzeugen von Temperatur und/oder Druck eingerichteten Aktoreinheit (202, 204) zwischen der Positivform (216) und einer ersten Silikonschicht (220) aus einem ersten Silikon mit einer die Aktoreinheit (202, 204) teilweise freilegenden Öffnung (218),
- Führen (154) einer Leitung (206) durch die Öffnung (218) der ersten Silikonschicht (220) zum Koppeln der Leitung (206) mit der Aktoreinheit (202, 204),
- Anordnen (156) einer zweiten Silikonschicht (222) aus einem zweiten Silikon auf einer der Positivform (216) abgewandten Seite der ersten Silikonschicht (220), wobei das zweite Silikon die Öffnung (218) der ersten Silikonschicht (220) und die Leitung (206) zumindest teilweise abdeckt und eine höhere Shore-Härte als das erste Silikon aufweist, und
- Vulkanisieren (158) der ersten Silikonschicht (220) und der zweiten Silikonschicht (222) mit der dazwischen angeordneten Leitung (206) unter Wärmezufuhr.

9. Verfahren nach Anspruch 8, wobei die Aktoreinheit (202, 204) ein Druckkissen umfasst, welches ein in ein drittes Silikon eingebettetes Stoffgewebe umfasst, wobei das dritte Silikon eine niedrigere Shore-Härte als das erste Silikon aufweist.

10. Verfahren nach einem der Ansprüche 8 oder 9, wobei die Leitung (206) ein Silikonschlauch ist, welcher ein Stoffgewebe an dem mit der Aktoreinheit (202, 204) gekoppelten Ende umfasst.

11. Verfahren nach einem der Ansprüche 8 bis 10, wobei die Leitung (206) ein von der ersten Silikonschicht (220) und/oder der zweiten Silikonschicht (222) freistehendes Ende zum Koppeln mit einer Steuereinheit (208) umfasst.

12. Verfahren nach einem der Ansprüche 8 bis 11, wobei vor dem Schritt des Vulkanisierens (158) der ersten Silikonschicht (220) und der zweiten Silikonschicht (222) mit der dazwischen angeordneten Leitung (206) ein Ende einer Manschette (224) in die erste Silikonschicht (220) und/oder die zweite Silikonschicht (222) eingearbeitet wird.

13. Mehrschichtiger HTV Silikonkörper (100) für eine Prothese oder einen Überzug der Prothese herstellbar nach einem Verfahren gemäß einem der Ansprüche 1 bis 7.

14. Mehrschichtiger Silikonliner (200) herstellbar nach einem Verfahren gemäß einem der Ansprüche 8 bis 12.

15. System (10) zur sensorischen Rückmeldung an einen Träger einer Prothese oder eines Überzugs der Prothese, umfassend:
einen mehrschichtigen HTV Silikonkörper (100) gemäß Anspruch 13 oder eine Prothese mit einem Silikonüberzug und einer zwischen der Prothese und dem Silikonüberzug angeordneten zum Erfassen einer Temperatur und/oder eines Drucks eingerichteten Sensoreinheit (102, 104) und
einen mehrschichtigen Silikonliner (200) gemäß Anspruch 14,
wobei der mehrschichtige HTV Silikonkörper (100) oder die Prothese und der mehrschichtige Silikonliner (200) dazu eingerichtet sind, derart in einem Datenaustausch zu stehen, dass eine durch die Sensoreinheit (102, 104) des mehrschichtigen HTV Silikonkörpers (100) oder der Prothese erfasste Temperatur und/oder ein erfasster Druck als Kommunikationssignal zum Antreiben der Aktoreinheit (202, 204) des mehrschichtigen Silikonliners (200) an diesen übermittelt wird.
